# EUROPÄISCHE PATENTSCHRIFT

(11) **EP 0 681 563 B1**
(45) Veröffentlichungstag und Bekanntmachung des Hinweises auf die Patenterteilung: **10.09.1997**
(21) Anmeldenummer: 94905675.8
(22) Anmeldetag: 21.01.1994
(51) Int. Cl.: C07C 17/02, C07C 19/045

(54) **VERFAHREN UND VORRICHTUNG ZUR HERSTELLUNG VON 1,2-DICHLORETHAN DURCH DIREKTCHLORIERUNG**
PROCESS AND DEVICE FOR PREPARING 1,2-DICHLORETHANE BY DIRECT CHLORINATION
PROCEDE ET DISPOSITIF POUR LA FABRICATION DE 1,2-DICHLORETHANE PAR CHLORATION DIRECTE

(30) Priorität: 27.01.1993 DE 4302177; 04.06.1993 DE 4318609
(43) Veröffentlichungstag der Anmeldung: 15.11.1995
(73) Patentinhaber: HOECHST AKTIENGESELLSCHAFT, 65926 Frankfurt am Main (DE)
(72) Erfinder: EICHLER, Jürgen, D-84556 Kastl (DE); KRUMBÖCK, Reinhard, D-84508 Burgkirchen (DE); KÖHN, Wenzel, D-84508 Burgkirchen (DE); SCHWARZMAIER, Peter, D-84556 Kastl (DE); WILD, Thomas, D-84508 Burgkirchen (DE); SPIELMANNLEITNER, Rudolf, D-84503 Altötting (DE); STÖGER, Manfred, D-84508 Burgkirchen (DE); MIELKE, Ingolf, D-84508 Burgkirchen (DE)
(86) Internationale Anmeldenummer: EP9400159
(87) Internationale Veröffentlichungsnummer: WO9417019

(56) Entgegenhaltungen:
- EP-A- 0 075 742
- DE-A- 4 103 281
- DE-A- 4 133 810
- US-A- 3 214 482
- US-A- 3 864 411

## Beschreibung

Bei dem sogenannten Direktchlorierungsverfahren wird 1,2-Dichlorethan, im folgenden EDC, durch Umsetzung von Ethylen mit Chlor gewonnen. Diese Additionsreaktion wird durch Metallhalogenide, die den Charakter einer Lewis-Säure haben, und ein Halogenid eines Metalls der ersten Hauptgruppe des periodischen Systems katalysiert.

Nach dem in der NL-A 6901398 beschriebenen Verfahren ist das Metallhalogenid mit Lewis-Säure-Charakter bevorzugt Eisen-(III)-chlorid. Das Metall der ersten Gruppe des Periodensystems hat bevorzugt eine Atomzahl von höchstens 12 (Natrium) und ist insbesondere Lithium. Das Molverhältnis des Halogenids mit Lewis-Säure-Charakter und des Halogenids des Metalls der ersten Gruppe des periodischen Systems liegt zwischen 1 : 2 und 2 : 1.

In der EP 0 111 203 B2 ist ein Verfahren beschrieben, bei dem als Katalysator ein wasserfreies Tetrachloroferrat, beispielsweise Natriumtetrachloroferrat, oder die entsprechenden Komponenten in stöchiometrischen Mengen eingesetzt werden. Nach der DE 41 03 281 Al wird als Katalysator Eisen-(III)-chlorid im Gemisch mit Natriumchlorid im molaren Verhältnis von 1 : 1,5 bis 1 : 2 eingesetzt.

Aus der DE-A-4 103 281 ist ein Verfahren zur Direktchlorierung in EDC als Lösungsmittel im Temperaturbereich von 30 bis 100 °C bekannt, das dadurch gekennzeichnet ist, daß das Eisen-(III)-chlorid im Gemisch mit Natriumchlorid im molaren Verhältnis von 1 : 1,5 bis 1 : 2 eingesetzt wird. In einem Vergleichsbeispiel soll dieses Molverhältnis 1 : 0,33 betragen, wobei jedoch relativ hohe Gehalte an dem unerwünschten Produkt 1,1,2-Trichlorethan erhalten werden.

Aus der EP-A-0 075 742 ist ein Verfahren und eine Vorrichtung zur Direktchlorierung bekannt, wobei die Reaktionsteilnehmer in das umlaufende flüssige Medium eingeleitet und nach intensiver Durchmischung in einer Mischzone das Gemisch in einer Reaktionszone bei etwa 75 bis 200 °C und etwa 1 bis 15 bar zur Reaktion gebracht werden. Aus der Reaktionszone wird ein Teil des flüssigen Reaktionsgemisches abgezogen und in zwei Teilströme aufgeteilt, von denen einer über einen Wärmeaustauscher in die Misch- und Reaktionszone zurückfließt, während der zweite einem Entspannungsgefäß zugeführt wird. Die Dämpfe aus diesem Entspannungsgefäß werden in eine Fraktionierkolonne eingeleitet, während der nichtverdampfte, flüssige Anteil in die Mischungs- und Reaktionszone zurückkehrt.

In der DE-A-4 133 810 [die einen Stand der Technik nach Artikel 54 (3) (4) darstellt] ist eine Vorrichtung zur Direktchlorierung vorgeschlagen, bei der der Reaktor mit einem Entspannungsbehälter verbunden ist, der Leitungen zur Ableitung des verdampften Produktes und zur Rückführung des unverdampften Produktes in den Reaktor enthält. Für diese vorgeschlagene Vorrichtung ist jedoch kein Lösebehälter vorgesehen.

Es wurde nun gefunden, daß die Direktchlorierung von Ethylen besonders vorteilhaft verläuft, wenn während der gesamten Reaktion das Molverhältnis von Natriumchlorid zu Eisen-(III)-chlorid im Bereich von 0,3 bis unter 0,5 gehalten wird. Das Einhalten dieser Bedingung erlaubt eine Reaktionsführung, bei der das EDC in einer so hohen Reinheit gewonnen wird, daß es ohne destillative Abtrennung hochsiedender Anteile unmittelbar in die Spaltung zu Vinylchlorid eingesetzt werden kann. Außerdem ist das erfindungsgemäße Verfahren in apparativer und energetischer Hinsicht wenig aufwendig. Bevorzugte Ausgestaltungen der Erfindung und die damit verbundenen Vorteile werden im folgenden näher erläutert.

Als Reaktionsmedium wird vorteilhaft flüssiges EDC verwendet, wobei die Reaktionsteilnehmer in das umgepumpte EDC eingespeist werden. Hierbei ist es nicht erforderlich, das Chlor in flüssiger Form einzusetzen, da das erfindungsgemäße Verfahren unter so schonenden Reaktionsbedingungen, insbesondere so niedrigen Drücken, ausgeführt werden kann, daß gasförmiges Chlor zum Einsatz kommen kann. So liegt der Druck vorteilhaft bei maximal 1 bar Überdruck, bevorzugt zwischen 0,4 und 0,6 bar Überdruck, der vorteilhaft mittels Inertgas-, vorzugsweise Stickstoffüberlagerung, eingestellt wird.

Die Reaktionstemperatur beträgt 50 bis 105 °C, vorteilhaft 70 bis 90 °C. Druck und Temperatur werden so aufeinander abgestimmt, daß die Reaktion unterhalb des Siedepunktes von EDC liegt.

In einer bevorzugten Ausgestaltung der Erfindung wird das EDC im ganzen oder vorzugsweise ein Teilstrom desselben in einen unter Vakuum stehenden Entspannungsbehälter geführt, wobei der Wärmeinhalt des EDC-Stromes zum Verdampfen eines Anteils des EDC führt. Das verdampfte EDC ist katalysatorfrei und weist nach Kondensation eine Reinheit von mindestens 99,9 % auf, so daß es unmittelbar der Spaltungsreaktion zu Vinylchlorid zugeführt werden kann. Die bei dem herkömmlichen Verfahren ohne Druckentspannung beziehungsweise EDC-Verdampfung notwendige Wasserwäsche zur Abtrennung des Katalysators sowie die anschließend erforderliche destillative Trocknung des EDC können entfallen.

Der Druck im Entspannungsbehälter beträgt zweckmäßig 0,2 bis 0,7 bar absolut. Dieser Unterdruck wird mit üblichen Gebläsen oder Pumpen, im folgenden Vakuumpumpe genannt, erzeugt und aufrechterhalten. Wenn diese Vakuumpumpe nicht auch zur Förderung des gasförmigen EDC dienen soll, wird dieses durch eine geeignete Vorrichtung, im folgenden als Kühler bezeichnet, kondensiert und in einem Behälter gesammelt.

Die Ausnützung der Reaktionswärme für die teilweise Verdampfung des EDC und die Führung der Reaktion im genannten bevorzugten Temperaturbereich bewirkt eine erhebliche Einsparung an Kühlmittel.

Der im Entspannungsbehälter nicht verdampfte, katalysatorhaltige EDC-Anteil wird in den EDC-Kreislauf zurückgeführt. Dies wird dadurch ermöglicht, daß die Reaktion nur einen sehr geringen Anteil an Nebenprodukten bildet und der EDC-Rückstrom nur unerhebliche Mengen an Chlorwasserstoff enthält.

Das Abgas ist praktisch chlorfrei. Die besonders bevorzugte Reaktionsführung, bei der nur ein - vorzugsweise relativ geringer - Teilstrom in den Entspannungsbehälter geleitet wird, hat weiterhin den Vorteil, daß ein Nachreaktor für die Umsetzung des restlichen Ethylens nicht notwendig ist. Vorrichtungen für dessen Abtrennung und Rückführung können aber auch bei dieser bevorzugten Ausgestaltung der Erfindung vorgesehen werden, da sie wenig aufwendig sind.

So kann man die Ethylen-Ausbeute weiter erhöhen beziehungsweise die Ethylen-Verluste weiter vermindern, indem man das ethylenhaltige Abgas aus dem Entspannungsbehälter nach der Vakuumpumpe mittels eines geeigneten Verdichters, bevorzugt eines Flüssigkeitsstrahlverdichters, der zweckmäßig am Boden des Reaktors angebracht ist, in die Reaktion zurückführt und das Ethylen erneut mit Chlor umsetzt. Als Treibstrahl für den bevorzugten Verdichter dient vorteilhaft ein Teilstrom des umgepumpten EDC.

Die Restmengen an Ethylen können aber auch in der Abgasverbrennung energetisch genutzt werden, vorteilhaft bei der Chlorwasserstoffrückgewinnung.

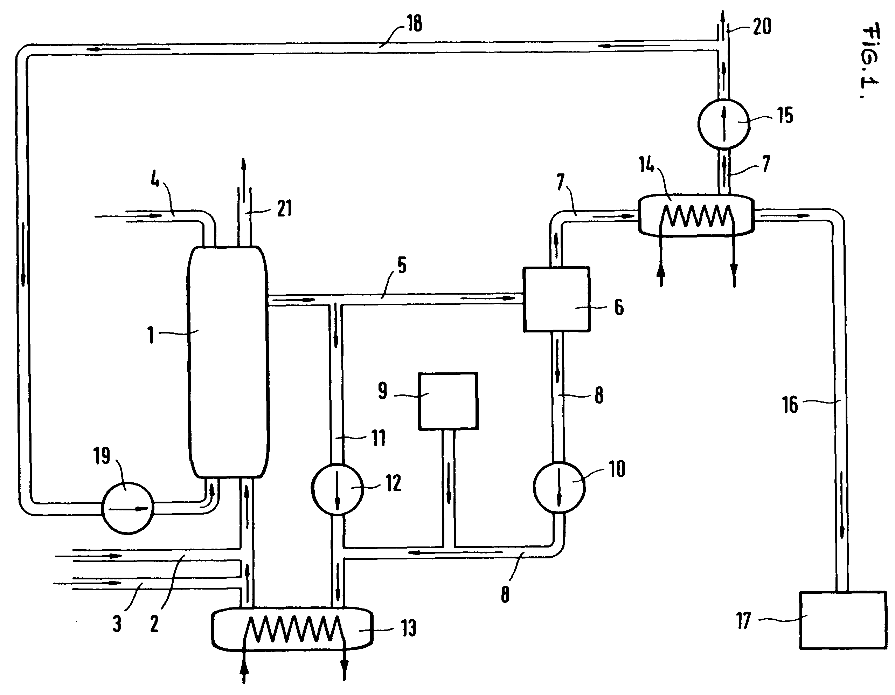

Die Erfindung betrifft deshalb auch eine Vorrichtung, die für das erfindungsgemäße Verfahren besonders geeignet ist. Sie ist - mit ihren bevorzugten Ausgestaltungen - in der Figur dargestellt.

Die erfindungsgemäße Vorrichtung enthält
einen Reaktor (1),
   Zuführungen (2) und (3) für Ethylen beziehungsweise Chlor sowie vorzugsweise (4) für ein Inertgas,
eine Leitung (5) zum
   Entspannungsbehälter (6), der unter Unterdruck steht und
eine Leitung (7) zur Ableitung des verdampften Produkts und
eine Leitung (8) zur Rückführung des unverdampften Produkts hat,
   an die vorzugsweise ein Lösebehälter (9) angeschlossen ist und die
   vorzugsweise über eine Pumpe (10) führt,
eine Leitung (11) zur Rückführung eines Produktteilstroms,
die vorzugsweise über eine Pumpe (12) und einen Kühler beziehungsweise Wärmetauscher (13) führt,
   in den Reaktor (1).

Die Leitung (7) für den EDC-Dampf führt zweckmäßig über einen Kühler (14) zur Vakuumpumpe (15). Vom Kühler (14) führt die Leitung (16) zum Sammelbehälter (17) für das verflüssigte EDC.

Vorteilhaft enthält die Vorrichtung weiterhin eine Leitung (18) von der (Druckseite der) Vakuumpumpe (15) über den Verdichter (19) zum Reaktor (1) zur Rückführug von Restmengen unumgesetzten Ethylens. (20) und (21) sind Leitungen zur Abgasentsorgung.

Wenn das Verfahren über längere Zeit läuft, ist dafür zu sorgen, daß das erfindungsgemäße Verhältnis von Natriumchlorid zu Eisen-(III)-chlorid aufrechterhalten bleibt. Wird also beispielsweise durch Korrosion Eisen-(III)-chlorid in die Anlage eingeschleppt, so muß, zweckmäßig aus dem Lösebehälter (9), entsprechend Natriumchlorid nachdosiert werden, da sonst die Reinheit des abdestillierten EDC zurückgeht beziehungsweise die Nebenproduktbildung ansteigt.

Das im Kreislauf geführte EDC hat im Mittel eine Reinheit von über 99 %, ist klar, das heißt es sind keine Feststoffpartikel erkennbar, und nur schwach gefärbt. Es ist deshalb normalerweise nicht erforderlich, einen Teilstrom zur Entfernung der Verunreinigungen abzuzweigen.

In den folgenden Beispielen wird die Erfindung näher erläutert.

### Beispiele 1 bis 4

Die Direktchlorierung wird in einem Reaktor (1) mit einem Volumen von 14,6 m³ und einem Füllstand von 81 Vol.-% durchgeführt. Der Druck wird über die Leitung (4) auf 0,54 bar Überdruck und die Temperatur bei 75 °C gehalten. Als Reaktionsmedium dient flüssiges EDC mit einem Eisen-(III)-chlorid-Gehalt von 700 ppm.

In das Reaktionsmedium wird über einen Lösebehälter (9) soviel Natriumchlorid eindosiert, bis das gewünschte Molverhältnis von Natriumchlorid zu Eisen-(III)-chlorid (siehe nachstehende Tabelle) erreicht ist. Dieses Molverhältnis wird während der gesamten Reaktion aufrechterhalten. In den Reaktor (1) werden pro Stunde über die Leitung (2) 2000 Nm³ Ethylen und über die Leitung (3) die entsprechende Menge gasförmiges Chlor eingeleitet. Ein Produktteilstrom wird über die Leitung (5) in einen nachgeschalteten Entspannungsbehälter (6) mit einem Druck von 0,3 bar absolut geführt. Hier werden pro Stunde 8,90 t EDC verdampft und über die Leitung (7) abgezogen. Dieses EDC ist katalysatorfrei und weist die in der Tabelle genannte Reinheit auf, die es erlaubt, daß das EDC unmittelbar zu Vinylchlorid verarbeitet werden kann. Der Gehalt an 1,1,2-Trichlorethan als Nebenprodukt ist ebenfalls in der folgenden Tabelle angegeben.

Das im Entspannungsbehälter (6) nicht verdampfte EDC wird über die Leitung (8) und eine Pumpe (10) in den Reaktor (1) zurückgeführt. Der Produkthauptstrom wird über die Leitung (11) und eine Pumpe (12) in die Leitung (8) eingespeist und - nötigenfalls über einen Kühler (13) - in den Reaktor (1) zurückgeführt.

In der Tabelle ist das Beispiel 1 ein Vergleichsbeispiel (ohne NaCl-Zusatz), die Beispiele 2 bis 4 sind erfindungsgemäß.

**Tabelle**

| | | | |
|---|---|---|---|
| Beispiel | Molverhältnis NaCl : FeCl₃ | Produkt: Gehalt an | |
| | | EDC [Gew.-%] | 1,1,2-Trichlorethan [Gew.-%] |
| 1 | 0 | 99,78 | 0,157 |
| 2 | 0,35 : 1 | 99,90 | 0,064 |
| 3 | 0,40 : 1 | 99,95 | 0,025 |
| 4 | 0,45 : 1 | 99,94 | 0,035 |

## Patentansprüche (Patentansprüche für folgende(n) Vertragsstaat(en): BE, ES, FR, GB, GR, IT, NL, SE)

1. Verfahren zur Herstellung von 1,2-Dichlorethan durch Umsetzung von Ethylen mit Chlor an einem Natriumchlorid-Eisen-(III)-chlorid-Katalysator, dadurch gekennzeichnet, daß während der gesamten Umsetzung das Molverhältnis von Natriumchlorid zu Eisen-(III)-chlorid im Bereich von 0,3 bis unter 0,5 bleibt.

2. Verfahren nach Anspruch 1, dadurch gekennzeichnet, daß das Molverhältnis von Natriumchlorid zu Eisen-(III)-chlorid im Bereich von 0,3 bis 0,45 liegt.

3. Verfahren nach Anspruch 1 oder 2, dadurch gekennzeichnet, daß die Umsetzung bei 50 bis 105 °C, vorzugsweise im Bereich 70 bis 90 °C, durchgeführt wird.

4. Verfahren nach einem oder mehreren der vorhergehenden Ansprüche, dadurch gekennzeichnet, daß die Umsetzung bei einem Druck von bis zu 1 bar, vorzugsweise bei 0,4 bis 0,6 bar, überdruck durchgeführt wird.

5. Verfahren nach Anspruch 4, dadurch gekennzeichnet, daß der Überdruck durch Inertgasüberlagerung eingestellt wird.

6. Verfahren nach einem oder mehreren der vorhergehenden Ansprüche, dadurch gekennzeichnet, daß das 1,2-Dichlorethan ganz oder teilweise in einen unter Unterdruck stehenden Behälter geleitet wird, wobei das abdestillierende Produkt abgetrennt und das restliche 1,2-Dichlorethan in den Prozeß zurückgeführt wird.

7. Verfahren nach einem oder mehreren der vorhergehenden Ansprüche, dadurch gekennzeichnet, daß als Reaktionsmedium im Kreislauf geführtes 1,2-Dichlorethan dient.

8. Verfahren nach einem oder mehreren der vorhergehenden Ansprüche, dadurch gekennzeichnet, daß das Chlor gasförmig eingespeist wird.

9. Vorrichtung zur Durchführung des Verfahrens nach einem oder mehreren der vorhergehenden Ansprüche, enthaltend einen Reaktor (1), Zuführungen (2) und (3) für Ethylen beziehungsweise Chlor, eine Leitung (5) zum Entspannungsbehälter (6), der eine Leitung (7) zur Ableitung des verdampften Produkts, eine Leitung (8) zur Rückführung des unverdampften Produkts hat, sowie eine Leitung (11) zur Rückführung eines Produktteilstroms in den Reaktor (1).

10. Vorrichtung nach Anspruch 9, zusätzlich gekennzeichnet durch eines oder mehrere der folgenden Merkmale:
eine Leitung (4) zum Reaktor (1) für ein Inertgas,
einen Lösebehälter (9), der an die Leitung (8) angeschlossen ist,
eine Pumpe (10) in der Leitung (8),
eine Pumpe (12) in der Leitung (11),
einen Kühler beziehungsweise Wärmetauscher (13) zwischen der Pumpe (12) und dem Reaktor (1),
in der Leitung (7) einen Kühler (14), von dem eine Leitung (16) zum EDC-Sammelbehälter (17) führt,
in der Leitung (7), nach dem Kühler (14), eine Vakuumpumpe (15),
eine Leitung (18) von der Vakuumpumpe (15) über einen Verdichter (19) zum Reaktor (1) und
Abgasleitungen (20) und (21).

## Patentansprüche (Patentansprüche für folgende(n) Vertragsstaat(en): DE)

1. Verfahren zur Herstellung von 1,2-Dichlorethan durch Umsetzung von Ethylen mit Chlor an einem Natriumchlorid-Eisen-(III)-chlorid-Katalysator, dadurch gekennzeichnet, daß während der gesamten Umsetzung das Molverhältnis von Natriumchlorid zu Eisen-(III)-chlorid im Bereich von 0,3 bis unter 0,5 bleibt.

2. Verfahren nach Anspruch 1, dadurch gekennzeichnet, daß das Molverhältnis von Natriumchlorid zu Eisen-(III)-chlorid im Bereich von 0,3 bis 0,45 liegt.

3. Verfahren nach Anspruch 1 oder 2, dadurch gekennzeichnet, daß die Umsetzung bei 50 bis 105 °C, vorzugsweise im Bereich 70 bis 90 °C, durchgeführt wird.

4. Verfahren nach einem oder mehreren der vorhergehenden Ansprüche, dadurch gekennzeichnet, daß die Umsetzung bei einem Druck von bis zu 1 bar, vorzugsweise bei 0,4 bis 0,6 bar, Überdruck durchgeführt wird.

5. Verfahren nach Anspruch 4, dadurch gekennzeichnet, daß der Überdruck durch Inertgasüberlagerung eingestellt wird.

6. Verfahren nach einem oder mehreren der vorhergehenden Ansprüche, dadurch gekennzeichnet, daß das 1,2-Dichlorethan ganz oder teilweise in einen unter Unterdruck stehenden Behälter geleitet wird, wobei das abdestillierende Produkt abgetrennt und das restliche 1,2-Dichlorethan in den Prozeß zurückgeführt wird.

7. Verfahren nach einem oder mehreren der vorhergehenden Ansprüche, dadurch gekennzeichnet, daß als Reaktionsmedium im Kreislauf geführtes 1,2-Dichlorethan dient.

8. Verfahren nach einem oder mehreren der vorhergehenden Ansprüche, dadurch gekennzeichnet, daß das Chlor gasförmig eingespeist wird.

9. Vorrichtung zur Durchführung des Verfahrens nach einem oder mehreren der vorhergehenden Ansprüche, enthaltend einen Reaktor (1), Zuführungen (2) und (3) für Ethylen beziehungsweise Chlor, eine Leitung (5) zum Entspannungsbehälter (6), der eine Leitung (7) zur Ableitung des verdampften Produkts, eine Leitung (8) zur Rückführung des unverdampften Produkts hat, einen Lösebehälter (9), der an die Leitung (8) angeschlossen ist, sowie eine Leitung (11) zur Rückführung eines Produktteilstroms in den Reaktor (1).

10. Vorrichtung nach Anspruch 9, zusätzlich gekennzeichnet durch eines oder mehrere der folgenden Merkmale:
eine Leitung (4) zum Reaktor (1) für ein Inertgas,
eine Pumpe (10) in der Leitung (8),
eine Pumpe (12) in der Leitung (11),
einen Kühler beziehungsweise Wärmetauscher (13) zwischen der Pumpe (12) und dem Reaktor (1),
in der Leitung (7) einen Kühler (14), von dem eine Leitung (16) zum EDC-Sammelbehälter (17) führt,
in der Leitung (7), nach dem Kühler (14), eine Vakuumpumpe (15),
eine Leitung (18) von der Vakuumpumpe (15) über einen Verdichter (19) zum Reaktor (1) und
Abgasleitungen (20) und (21).

## Claims (Claims for the following Contracting State(s): BE, ES, FR, GB, GR, IT, NL, SE)

1. A process for preparing 1,2-dichloroethane by reaction of ethylene with chlorine in the presence of a sodium chloride-iron(III) chloride catalyst, which comprises maintaining the molar ratio of sodium chloride to iron(III) chloride in the range from 0.3 to below 0.5 during the whole reaction.

2. The process as claimed in claim 1, wherein the molar ratio of sodium chloride to iron(III) chloride is in the range from 0.3 to 0.45.

3. The process as claimed in claim 1 or 2, wherein the reaction is carried out at from 50 to 105°C, preferably in the range from 70 to 90°C.

4. The process as claimed in one or more of the preceding claims, wherein the reaction is carried out at a pressure of up to 1 bar gauge pressure, preferably at from 0.4 to 0.6 bar gauge pressure.

5. The process as claimed in claim 4, wherein the gauge pressure is set by inert gas blanketing.

6. The process as claimed in one or more of the preceding claims, wherein all or part of the 1,2-dichloroethane is passed to a container maintained at reduced pressure, the product which distills off being separated off and the remaining 1,2-dichloroethane being returned to the process.

7. The process as claimed in one or more of the preceding claims, wherein the reaction medium is circulating 1,2-dichloroethane.

8. The process as claimed in one or more of the preceding claims, wherein the chlorine is fed in as gas.

9. Apparatus for carrying out the process as claimed in one or more of the preceding claims, comprising a reactor (1), feed lines (2) and (3) for ethylene and chlorine, respectively, a line (5) to the expansion vessel (6) which has a line (7) for removing the vaporized product and a line (8) for returning the unvaporized product, and a line (11) for returning a product partial stream to the reactor (1).

10. Apparatus as claimed in claim 9, wherein one or more of the following features are also present:
a line (4) to the reactor (1) for an inert gas,
a dissolving vessel (9), which is connected to line (8),
a pump (10) in line (8),
a pump (12) in line (11),
a cooler or heat exchanger (13) between the pump (12) and the reactor (1),
in line (7) a cooler (14) from which a line (16) leads to the EDC-receiver (17),
in line (7) downstream of the cooler (14) a vacuum pump (15),
a line (18) from the vacuum pump (15) via a compressor (19) to the reactor (1) and
waste gas lines (20) and (21).

## Claims (Claims for the following Contracting State(s): DE)

1. A process for preparing 1,2-dichloroethane by reaction of ethylene with chlorine in the presence of a sodium chloride-iron(III) chloride catalyst, which comprises maintaining the molar ratio of sodium chloride to iron(III) chloride in the range from 0.3 to below 0.5 during the whole reaction.

2. The process as claimed in claim 1, wherein the molar ratio of sodium chloride to iron(III) chloride is in the range from 0.3 to 0.45.

3. The process as claimed in claim 1 or 2, wherein the reaction is carried out at from 50 to 105°C, preferably in the range from 70 to 90°C.

4. The process as claimed in one or more of the preceding claims, wherein the reaction is carried out at a pressure of up to 1 bar gauge pressure, preferably at from 0.4 to 0.6 bar gauge pressure.

5. The process as claimed in claim 4, wherein the gauge pressure is set by inert gas blanketing.

6. The process as claimed in one or more of the preceding claims, wherein all or part of the 1,2-dichloroethane is passed to a container maintained at reduced pressure, the product which distills off being separated off and the remaining 1,2-dichloroethane being returned to the process.

7. The process as claimed in one or more of the preceding claims, wherein the reaction medium is circulating 1,2-dichloroethane.

8. The process as claimed in one or more of the preceding claims, wherein the chlorine is fed in as gas.

9. Apparatus for carrying out the process as claimed in one or more of the preceding claims, comprising a reactor (1), feed lines (2) and (3) for ethylene and chlorine, respectively, a line (5) to the expansion vessel (6) which has a line (7) for removing the vaporized product and a line (8) for returning the unvaporized product, a dissolving vessel (9), which is connected to line (8), and a line (11) for returning a product partial stream to the reactor (1).

10. Apparatus as claimed in claim 9, wherein one or more of the following features are also present:
a line (4) to the reactor (1) for an inert gas,
a pump (10) in line (8),
a pump (12) in line (11),
a cooler or heat exchanger (13) between the pump (12) and the reactor (1),
in line (7) a cooler (14) from which a line (16) leads to the EDC-receiver (17),
in line (7) downstream of the cooler (14) a vacuum pump (15),
a line (18) from the vacuum pump (15) via a compressor (19) to the reactor (1) and
waste gas lines (20) and (21).

## Revendications (Revendications pour l'(les) Etat(s) contractant(s) suivant(s): BE, ES, FR, GB, GR, IT, NL, SE)

1. Procédé de préparation du 1,2-dichloréthane par réaction de l'éthylène avec le chlore sur un catalyseur de chlorure de sodium-chlorure de fer(III), caractérisé en ce que le rapport molaire de chlorure de sodium au chlorure de fer(III) pendant la totalité de la réaction est maintenu dans l'intervalle de 0,3 à une valeur au-dessous de 0,5.

2. Procédé selon la revendication 1, caractérisé en ce que le rapport molaire de chlorure de sodium au chlorure de fer(III) est dans l'intervalle de 0,3 à 0,45.

3. Procédé selon la revendication 1 ou 2, caractérisé en ce que l'on met en oeuvre la réaction à une température dans l'intervalle de 50 à 105°C, de préférence de 70 à 90°C.

4. Procédé selon une ou plusieurs des revendications précédentes, caractérisé en ce que l'on met en oeuvre la réaction à une pression allant jusqu'à 1 bar, de préférence à une surpression de 0,4 à 0,6 bar.

5. Procédé selon la revendication 4, caractérisé en ce que l'on établit la surpression par superposition de gaz inerte.

6. Procédé selon une ou plusieurs des revendications précédentes, caractérisé en ce que l'on conduit le 1,2-dichloréthane entièrement ou partiellement dans un récipient dans lequel règne un défaut de pression, le produit distillant étant séparé et le reste de 1,2-dichloréthane étant recyclé au processus.

7. Procédé selon une ou plusieurs des revendications précédentes, caractérisé en ce que le 1,2-dichloréthane amené au circuit sert de milieu réactionnel.

8. Procédé selon une ou plusieurs des revendications précédentes, caractérisé en ce que l'on apporte le chlore sous forme gazeuse.

9. Installation pour la mise en oeuvre du procédé selon une ou plusieurs des revendications précédentes, comportant un réacteur (1), des conduites d'amenée (2) et (3) pour l'éthylène respectivement le chlore, une conduite (5) allant vers le ballon de détente (6), qui comporte une conduite (7) pour l'évacuation du produit évaporé, une conduite (8) pour le recyclage du produit n'ayant pas été évaporé, ainsi qu'une conduite (11) pour le recyclage d'un flux partiel de produit au réacteur (1).

10. Installation selon la revendication 9, caractérisée en plus par une ou plusieurs caractéristiques suivantes :
une conduite (4) vers le réacteur (1) pour un gaz inerte,
un réservoir de solutions (9) relié à la conduite (8), une pompe (10) dans la conduite (8),
une pompe (12) dans la conduite (11),
un refroidisseur, respectivement échangeur de chaleur (13), entre la pompe (12) et le réacteur (1), dans la conduite (7) un refroidisseur (14) depuis lequel mène une conduite (16) au bac de collecte de EDC (17),
dans la conduite (7), en amont du refroidisseur (14), une pompe à vide (15),
une conduite (18) allant de la pompe à vide (15) par un compresseur (19) vers le réacteur (1) et des conduites d'évacuation des effluents gazeux (20) et (21).

## Revendications (Revendications pour l'(les) Etat(s) contractant(s) suivant(s): DE)

1. Procédé de préparation du 1,2-dichloréthane par réaction de l'éthylène avec le chlore sur un catalyseur de chlorure de sodium-chlorure de fer(III), caractérisé en ce que le rapport molaire de chlorure de sodium au chlorure de fer(III) pendant la totalité de la réaction est maintenu dans l'intervalle de 0,3 à une valeur au-dessous de 0,5.

2. Procédé selon la revendication 1, caractérisé en ce que le rapport molaire de chlorure de sodium au chlorure de fer(III) est dans l'intervalle de 0,3 à 0,45.

3. Procédé selon la revendication 1 ou 2, caractérisé en ce que l'on met en oeuvre la réaction à une température dans l'intervalle de 50 à 105°C, de préférence de 70 à 90°C.

4. Procédé selon une ou plusieurs des revendications précédentes, caractérisé en ce que l'on met en oeuvre la réaction à une pression allant jusqu'à 1 bar, de préférence à une surpression de 0,4 à 0,6 bar.

5. Procédé selon la revendication 4, caractérisé en ce que l'on établit la surpression par superposition de gaz inerte.

6. Procédé selon une ou plusieurs des revendications précédentes, caractérisé en ce que l'on conduit le 1,2-dichloréthane entièrement ou partiellement dans un récipient dans lequel règne un défaut de pression, le produit distillant étant séparé et le reste de 1,2-dichloréthane étant recyclé au processus.

7. Procédé selon une ou plusieurs des revendications précédentes, caractérisé en ce que le 1,2-dichloréthane amené au circuit sert de milieu réactionnel.

8. Procédé selon une ou plusieurs des revendications précédentes, caractérisé en ce que l'on apporte le chlore sous forme gazeuse.

9. Installation pour la mise en oeuvre du procédé selon une ou plusieurs des revendications précédentes, comportant un réacteur (1), des conduites d'amenée (2) et (3) pour l'éthylène respectivement le chlore, une conduite (5) allant vers le ballon de détente (6), qui comporte une conduite (7) pour l'évacuation du produit évaporé, une conduite (8) pour le recyclage du produit n'ayant pas été évaporé, un réservoir de solutions (9) relié à la conduite (8), ainsi qu'une conduite (11) pour le recyclage d'un flux partiel de produit au réacteur (1).

10. Installation selon la revendication 9, caractérisée en plus par une ou plusieurs caractéristiques suivantes :
une conduite (4) vers le réacteur (1) pour un gaz inerte,
une pompe (10) dans la conduite (8),
une pompe (12) dans la conduite (11),
un refroidisseur, respectivement échangeur de chaleur (13), entre la pompe (12) et le réacteur (1), dans la conduite (7) un refroidisseur (14) depuis lequel mène une conduite (16) au bac de collecte de EDC (17),
dans la conduite (7), en amont du refroidisseur (14), une pompe à vide (15),
une conduite (18) allant de la pompe à vide (15) par un compresseur (19) vers le réacteur (1) et des conduites d'évacuation des effluents gazeux (20) et (21).
